# NEUE EUROPÄISCHE PATENTSCHRIFT

(11) **EP 1 411 997 B2**
(45) Veröffentlichungstag und Bekanntmachung des Hinweises auf die Entscheidung über den Einspruch: **27.03.2019**
(45) Hinweis auf die Patenterteilung: 31.08.2005
(21) Anmeldenummer: 02754251.3
(22) Anmeldetag: 25.06.2002
(51) Int. Cl.: A61L 31/04

(54) **TEXTILES IMPLANTAT MIT MONOFILEN POLYVINYLFLUORID-FÄDEN**
TEXTILE IMPLANT MADE OF MONOFIL THREADS FROM POLYVINYL FLUORIDE
IMPLANT TEXTILE A BASE DE MONOFIL EN FLOURURE DE POLYVINYLE

(30) Priorität: 26.06.2001 DE 10130289; 10.07.2001 DE 10132762
(43) Veröffentlichungstag der Anmeldung: 28.04.2004
(73) Patentinhaber: FEG Textiltechnik Forschungs- und Entwicklungsgesellschaft MbH, 52070 Aachen (DE)
(72) Erfinder: KLINGE, Uwe, B-4850 Montzen (BE); KLOSTERHALFEN, Bernd, 52134 Herzogenrath (DE); OBOLENSKI, Boris, 52070 Aachen (DE); SCHNEEMELCHER, Stefan, 52064 Aachen (DE)
(74) Vertreter: Gottschald, Jan
(86) Internationale Anmeldenummer: PCT/DE2002/002287
(87) Internationale Veröffentlichungsnummer: WO 2003/002167

(56) Entgegenhaltungen:
- WO-A-98/37813
- WO-A2-02/19944
- DE-U- 8 911 028
- US-A- 5 522 879
- JUNGE, K. ET AL: "ELASTICITY OF THE ANTERIOR ABDOMINAL WALL AND IMPACT FOR REPARATION OF INCISIONAL HERNIAS USING MESH IMPLANTS", HERNIA, vol. 5, 2001, pages 113-118,

## Beschreibung

Die Erfindung betrifft ein textiles Implantat.

In der Medizintechnik, insbesondere in der Chirurgie, stellt sich häufig die Aufgabe, schadhafte Hohlräume oder Spalten im menschlichen oder tierischen Körperstamm mittels Platzhaltern zu stabilisieren und/oder schadhafte Öffnungen nachhaltig zu verschließen.

So ist es beispielsweise bei der Operation von Bauch- und Leisten-Hernien oftmals erforderlich, den für die Operation geöffneten Körperstamm mit Hilfe einer Naht zu verschließen. Eine einfache konventionelle Naht ist jedoch z. B. beim Husten hohen mechanischen Beanspruchungen ausgesetzt und kann dadurch leicht gefährdet werden. Deshalb wird in solchen Fällen oft ein maschenförmiges Implantat (Mesh) zur mechanischen Stützung der Naht in die Bauchwand eingesetzt. Im Körper des Patienten können diese Meshes in zwei oder mehr Richtungen Kräfte aufnehmen und entlasten hierdurch die eigentliche Naht.

Handelsübliche Meshes bestehen derzeit überwiegend aus multifilen Polymeren, insbesondere Polyester und Polypropylen verschiedener Struktur.

Leider treten bei der Verwendung von Implantaten dieser Art häufig postoperative Komplikationen auf. Mit fortschreitender Zeit wird die Flexibilität des Implantats geringer, der Stoff verhärtet und verursacht eine entzündliche Fremdkörperreaktion im menschlichen Körper mit nachfolgender aggressiver Vemarbung. Die Beweglichkeit des Patienten wird hierdurch dauerhaft eingeschränkt und/oder schmerzlich behindert.

Zudem wird das Implantat vom Körpergewebe eingekapselt und verbleibt somit quasi als Fremdkörper in der reparierten Struktur. Polypropylen und Polyester sind zwar in der Chirurgie zugelassene Werkstoffe, binden sich aber nicht in den Körper ein. Diese Stoffe werden vom Körper dauerhaft als Fremdkörper identifiziert und ein homogenes Einwachsen in das menschliche Gewebe hierdurch verhindert.

Die DE 89 11 028 U offenbart chirurgische Nahtmaterialien in Form geflochtener Bänder aus Monofilen von Polyvinylidenflourid (PVDF). Das geflochtene Band ist dazu vorgesehen, zur Unterstützung von Bändern im Gelenkbereich bei Menschen eingesetzt zu werden.

Der Erfinder hat sich die Aufgabe gestellt, ein Implantat zu entwickeln, das die genannten nachteilhaften Eigenschaften nicht oder zumindest in geringerem Umfang aufweist und somit eine bessere Verträglichkeit für den Menschen verspricht.

Diese Aufgabe löst ein textiles Implantat mit monofilen Fäden aus Polyvinylidenflourid (PVDF) und mit einer netzförmigen Flächenstruktur gemäß Anspruch 1.

Eine besonders vorteilhafte Eignung weist das erfindungsgemäße Implantat dadurch auf, dass es als Flächenstruktur ausgebildet ist. Die Flächenstruktur ermöglicht es dem Operateur in besonderem Maße, an mehreren Stellen potentielle Anschlussstellen für Nähte vorzusehen und auftretende Kräfte flächig abzutragen. Zudem können auch weiter von der eigentlichen Schadstelle entfernte Körperteile mit dem erfindungsgemäßen Implantat zur Befestigung erreicht werden, ohne dass hierfür übermäßig viel Platz zwischen den körpereigenen Gewebestrukturen benötigt wird.

Einen besonders großen Vorteil erfährt das erfindungsgemäße Implantat dadurch, dass seine Flächenstruktur netzförmig ausgebildet ist. Diese Ausführungsart weist zu den beschriebenen Vorteilen der rein flächigen Struktur außerdem den Vorteil auf, dass körpereigenes Gewebe in die Netzwaben leicht einwachsen und das Implantat so möglichst gut in den Körper integrieren kann.

Polyvinylidenfluorid (PVDF) ist eine Stoffklasse mit für den hier beschriebenen Einsatz sehr vorteilhaften biologischen Eigenschaften. Insbesondere im direkten Vergleich mit Polyester hat es eine deutlich bessere Hydrolysebeständigkeit. Während Polypropylen mit fortschreitender Zeit an Flexibilität einbüßt und der Stoff somit verhärtet, tritt dieses Phänomen beim PVDF nicht auf, somit auch nicht die hiermit einhergehende Einschränkung der

Bewegungsfreiheit des Patienten. Von PVDF sind keine Alterungsprozesse bekannt.

Zudem sind die textilen Eigenschaften von PVDF innerhalb eines Temperaturspektrums von - 40 °C bis + 160 °C stabil. Das Implantat wird im Körper eines Patienten im Normalfall keine Temperatur außerhalb dieses Spektrums erfahren. Die Reibfestigkeit liegt auf dem Niveau der Polyamide und übersteigt damit beträchtlich diejenige der Polyester. PVDF zeigt außerdem eine hohe Beständigkeit gegenüber vielen organischen Säuren und Mineralsäuren sowie gegenüber aliphatischen und aromatischen Kohlenwasserstoffen, Alkoholen und halogenierten Lösungsmitteln. Im Vergleich zu Polypropylen ist außerdem die entzündliche Fremdkörperreaktion des menschlichen Gewebes deutlich reduziert.

Durch die Verwendung von monofilen Fäden kann darüber hinaus noch die Gesamtzahl der das Implantat zusammensetzenden Fäden reduziert werden. Hierdurch wird eine vorteilhafte Reduzierung der Gesamtfadenoberfläche und somit der gesamten Implantatoberfläche erzielt.

In einer vorteilhaften Ausführung der Erfindung besteht das textile Implantat überwiegend oder sogar vollständig aus monofilen Fäden. Hierdurch treten die vorteilhaften Eigenschaften der Monofilstruktur noch stärker in den Vordergrund.

Vorschlagsgmäß weist das erfindungsgemäße Implantat eine poröse Struktur auf. Hierdurch kann sich das nach- oder anwachsende menschliche Gewebe in die Poren ausbreiten und somit nicht nur bereits anfänglich verbesserte mechanische Eigenschaften erzielen, sondern auch zu einer besseren Gesamtintegration des Implantats in die vorhandene Gewebestruktur beitragen. Die Abkapselung des Implantats, die oft zu beobachten ist, wird somit weitestgehend oder sogar vollständig vermieden.

Von besonderem Vorteil ist es hierbei, wenn das erfindungsgemäße Implantat eine Porengröße von 1 bis 5 mm, vorzugsweise 1 bis 3 mm, hat. Es hat sich herausgestellt, dass bei einer Porenstruktur mit diesen Größen die beschriebenen vorteilhaften Mechanismen in besonders hohem Maße stattfinden.

Das erfindungsgemäße Implantat weist ein Gewebe, Gewirk, Gestrick oder Gelege auf. Mit diesen Konstruktionsarten der monofilen Fäden ist gewährleistet, dass auftretende Kräfte möglichst gut innerhalb des erfindungsgemäßen Implantats aus mehreren Richtungen aufgenommen und in mehrere Richtungen abgetragen werden können. Zudem gibt ein so gestaltetes Implantat dem Operateur die Möglichkeit, Nähte leicht anschließen zu können und so mit größtmöglicher Entscheidungsfreiheit Anschlüsse vom Implantat zum benachbarten Gewebe herstellen zu können.

Besonders gute Ergebnisse können dadurch erzielt werden, dass das erfindungsgemäße Implantat eine flexible Struktur aufweist. Hier sind mehrere Vorteile zu nennen: zum einen kann der Patient ein flexibles erfindungsgemäßes Implantat nur in geringerem Maß sensorisch wahrnehmen, da sich auch das körpereigene Gewebe um das Implantat herum elastisch verhält und somit eine Homogenität der Elastizität im versorgten Bereich besser gewährleistet werden kann. Zum anderen wird hierdurch auch die Gefahr einer Abscherung oder eines Abreißens des erfindungsgemäßen Implantats vom angenähten Gewebe deutlich verringert, da Spannungsspitzen besser abgetragen werden können. Hiermit reduziert sich gleichzeitig auch die Wahrscheinlichkeit einer weiteren erforderlichen Operation.

Zur gezielten Einsetzbarkeit bei unterschiedlichen medizinischen Erfordernissen und/oder Zielsetzungen kann es auch vorteilhaft sein, wenn zumindest ein Teil des erfindungsgemäßen Implantats ein resorbierbares Material aufweist.

Weiterhin ist es so, dass zumindest ein Teil des Implantats ein biokompatibles Material aufweist. Unabhängig von der mechanischen Kompatibilität des verwendeten Implantats ist als weiterer Faktor die biologische Kompatibilität ausschlaggebend für die erfolgreiche Integration des Implantats in das körpereigene Gewebe. Entscheidend ist hier die Biokompatibilität der Implantatoberfläche. Unmittelbar nach dem Einsetzen des Implantats in den Körperstamm findet eine Vielzahl von Reaktionen zwischen Implantat und Gewebe statt. Die erste Reaktion ist hierbei physikalischer Natur: Biomoleküle, insbesondere Proteine, werden auf der Oberfläche des Implantats unkontrolliert absorbiert. Diese absorptiv gebundenen Biomoleküle scheinen konformativ so verändert zu sein, dass sie ihre biologische Aktivität verlieren oder eine andersgeartete unge wollte biologische Funktion erfüllen.

Durch die Konformation der absorbierten Proteine werden nun die Zelladhäsion und die Zellausbreitung auf der Oberfläche des Implantats entscheidend beeinflusst. Beispielsweise sollen einzelne Proteinmoleküle entweder durch gewollte Konformationsänderungen zu Signalstoffen umgewandelt werden, oder aber als Signalstoffe wirkende Proteinfragmente werden durch katalytische (proteolytische) Reaktionen freigesetzt. Die Biokompatibilität des Implantats hängt somit in besonderem Maße davon ab, die Proteinabsorption zielgerichtet zu beeinflussen.

Durch die gezielte Einführung biokompatibler, funktioneller Gruppen in das Material werden spezifische Ligand-Rezeptor-Wechselwirkungen, wie sie zwischen Zellen und extrazellulärer Matrix (ECM) selber ablaufen, initiiert. Durch die Verwendung geeigneter Liganden zur Adressierung von Oberflächenrezeptoren der körpereigenen Zellen wird die Reaktion des Organismus' auf das Implantat beeinflussbar. Hierdurch wird eine aktive Integration ermöglicht. Idealerweise kommt es sogar zu einer Pennanentintegration in das menschliche Gewebe.

Das Implantat kann so ausgebildet sein, dass es zumindest auf seiner Ober- und/oder seiner Unterseite ein- oder mehrstückig mit einem Netz aus biokompatiblem Material versehen ist. Auf diese Weise kann eine starke Stützung des umgebenden Körpergewebes erreicht werden.

Besonders gute Ergebnisse erzeugt das erfindungsgemäße Implantat auch, wenn zumindest ein Teil des Implantats eine Beschichtung aufweist. Da zumindest die anfänglichen Reaktionen zwischen Implantat und Körpergewebe reine Oberflächenreaktionen sind, können durch eine Oberflächenbeschichtung des Implantats die hier ablaufenden Prozesse gezielt beeinflusst werden. Derartige Beschichtungen sind bereits bekannt. Beispielsweise können durch Zelladhäsionsproteine die Biokompatibilität verbessert oder auch durch andere Beschichtungsarten, zum Beispiel mit Medikamenten, Infekte und andere Komplikationen in der postoperativen Phase gemindert oder sogar verhindert werden.

Um der Elastizität des uzngebenden Gewebes und somit dem körpereigenen Verhalten Rechnung tragen zu können, ist es so, dass sich das erfindungsgemäße Implantat bei einer Zugbelastung in einer Hauptdehnungsrichtung bei Fₘₐₓ = 16 N/cm um 30 bis 40 % und in einer anderen Hauptrichtung bei Fₘₐₓ= 32N/cm um 15 bis 25 % dehnt.

Dadurch, dass in mindestens einer Hauptdehnungsrichtung das Dehnungsverhalten unterschiedlich ist, kann zum Beispiel erreicht werden, dass das Implantat dort, wo es in unmittelbarer Nähe zu einem Knochen verläuft, eine angepasst geringe Dehnung hat.

Hierbei sind an dem Implantat mindestens zwei winkehnäßig zueinander versetzt laufende Hauptdehnungsrichtungen definiert. Das Dehnungsverhalten in den Hauptdehnungsrichtungen wird durch Fadenabstände und/oder Fadenstärke und/oder Fadenmaterial und/oder Maschenweiten bestimmt. Es kann zweckmäßig sein, dass die beiden Hauptdehnungsrichtungen normal zueinander vorgesehen oder aber auch weitere Hauptdehnungsrichtungen vorgesehen sind.

In einer vorteilhaften Ausführung der Erfindung ist zumindest eine der Hauptdelmungsrichtungen des vorgestellten Implantats durch mindestens einen unterscheidbaren Faden und/oder farblich erkennbar. Handelsübliche Meshes haben zwar unterschiedliche insbesondere mechanische Eigenschaften in den verschiedenen Dehnungsrichtungen. Diese sind jedoch an den Meshes nicht als solche kenntlich gemacht und daher für den Chirurgen nicht absehbar. Er kann zwar ein Mesh in der Größe dem Defekt des Patienten anpassen und es dann in den Körperstamm implantieren, hat jedoch nicht die Möglichkeit, die Orientierung des Meshes so zu bestimmen, dass dieses den auftretenden dynamometrischen Gegebenheiten der reparierten Stelle bestmöglich mechanisch angepasst ist. Die Einführung einer solchen Markierung ermöglicht es dem operierenden Arzt nicht nur, das einzusetzende Stück auch in Bezug auf die unterschiedlichen Dehnungsverhalten in den verschiedenen Hauptdehnungsrichtungen gezielt auszuwählen und somit das Implantat den elastischen Eigenschaften des umgebenden Körpergewebes oder gemäß einem angestrebten elastischen Verhalten möglichst gut anpassen zu können. Er kann sogar symmetrische oder mehrfach symmetrische Implantate (also insbesondere auch mit runden, quadratischen, usw. Formen) gezielt ausrichten.

Zudem ist eine solche Markierung von Vorteil, wenn ein einzusetzendes Implantat während der Operation durch mehrere Hände geht und mittels der erfindungsgemäßen Kennzeichnung eine größere Transparenz und somit Kontrollierbarkeit für alle an der Operation beteiligten Personen geschaffen wird.

Besonders vorteilhaft ist es, wenn das erfindungsgemäße Implantat vorstehende Anschlusselemente aufweist. Mit diesen Anschlusselementen kann der operierende Arzt besonders leicht Anschlüsse an das die Wunde umgebende oder das als Befestigungspunkt vorgesehene Körpergewebe herstellen, ohne dass er weiterer Hilfskonstruktionen bedarf, mittels derer er das Implantat an die vorgesehenen Ankerpunkte anschließen kann. Dadurch, dass die Anschlusselemente vorstehen, kann die Form des Implantats weiterhin völlig unabhängig von der Wahl der Anschlusspunkte auf die Abmessungen der zu schließenden Wunde abgestimmt werden. Gleichzeitig können die Ankerpunkte unabhängig von Größe und Lage der eigentlichen Schadstelle ausgewählt werden.

Es ist beispielsweise möglich, dass das Implantat zumindest auf seiner Ober- und/oder Unterseite seitlich abstehende Anschlussabschnitte aufweist. Diese Anschlussabschnitte können an den Grundkörper des Implantats angeformt sein und das anschließende Gewebe zumindest teilweise übergreifen.

Die größenmäßige Anpassung des Implantats an die gegebenen Anforderungen erfolgt beispielsweise dadurch, dass das Implantat entsprechend zuschneidbar ist.

In einer besonders vorteilhaften Ausführung des erfindungsgemäßen Implantats zeichnet sich dieses dadurch aus, dass es einen Körper und eine Flächenstruktur aufweist. In dieser Form übernimmt der Körper hauptsächlich die Aufgabe, die Klaffung der eigentlichen Körperöffnung möglichst gut zu füllen und zu stützen, während der flächige Teil die Anschließbarkeit an und die Integrierbarkeit in das Körpergewebe gezielt fördert.

Ein alloplastisches Implantat der vorgeschlagenen Art kann sich zudem auch dadurch auszeichnen, dass es ergänzend zu PVDF ein Material aus der Gruppe folgender Materialien und/oder derer Derivate aufweist: Polymer auf der Basis von Polyester, Polyglykolsäure, Polytetrafluorethylen, Polyvinyl, Polyamid, Polypropylen, Polyethylen, Elastan, Polyurethan, Polyvinylalkohol, Polylactid, Polyglycolid, Polydioxanon, Alginat, Kasein, Protein, Lactid/Glycolid-copolymere und andere Copolymere der aufgeführten Materialien.

Vorteilhafte Ausführungsformen und eine vorteilhafte Anwendung der Erfindung sind nachstehend anhand der Zeichnungen beschrieben.

Es zeigt:
- Figur 1: eine räumliche Ansicht eines beispielhaften Implantats,
- Figur 2: die räumliche Ansicht eines kleinen Ausschnitts aus einem flächigen Implantatstück und
- Figur 3: in einer Prinzipskizze den Schnitt durch eine reparierte Bauchwand unter Verwendung eines vorgeschlagenen Implantats.

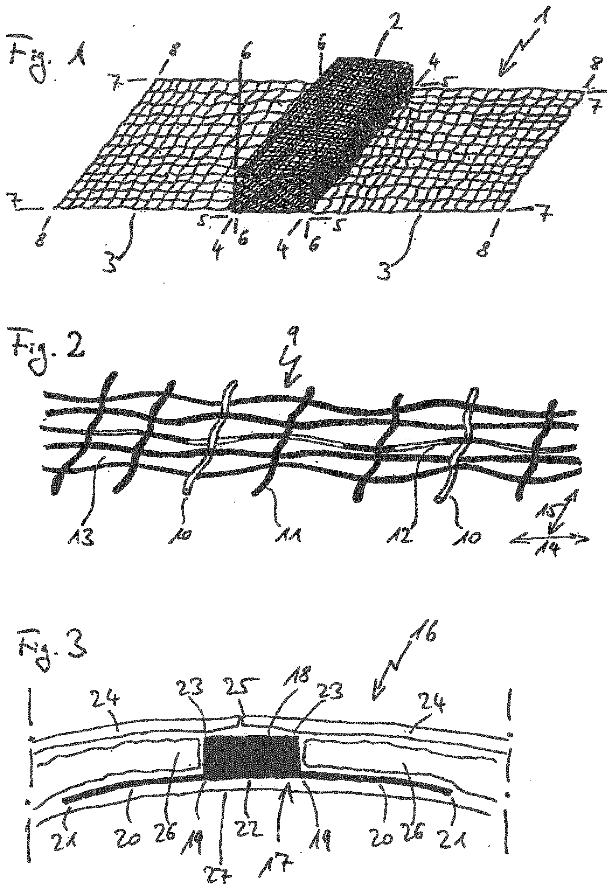

Das Implantat 1 in Figur 1 besteht aus einem körperhaften, strangförmigen Element 2 sowie zwei seitlich abstehenden flächigen Implantatstücken 3, die an zwei Kanten 4 des körperhaften Stücks befestigt sind. Sowohl die flächigen Elemente 3 als auch der körperhafte Teil 2 des Implantats 1 sollen im vorliegenden Beispiel überwiegend aus monofilen PVDF-Fäden bestehen, z. B. sogar aus identischem Material. Die flächigen Elemente 3 sind hierbei als Netzstruktur ausgebildet und sollen als vorstehende Anschlusselemente zur Verbindung des Implantats 1 mit ausgewählten Körperstellen mittels Nähten oder Ähnlichem fungieren. Die Längen der Kanten 4, 5 und 6 des Körpers 2 im gezeigten Ausführungsbeispiel betragen jeweils mehr als einen Zentimeter; die Längen der Kanten 7 und 8 der flächigen Anschlusselemente 3 betragen im Beispiel jeweils mehrere Zentimeter. Der körperhafte Teil 2 erfüllt bei dieser Ausführungsform hauptsächlich die Funktion, den Spalt im menschlichen Körperstamm auszufüllen und zu stützen.

In Figur 2 ist eine beispielhafte Ausführungsform eines flächenhaften netzfönnigen Implantatelements in einem Detailausschnitt zu sehen. Der zu sehende Ausschnitt 9 kann zum Beispiel ein wahlloser, nahezu rechteckiger Ausschnitt aus den flächigen Anschlusselementen 3 der vorausgegangenen Figur 1 sein. Deutlich zu sehen ist hier die Netzstruktur aus den Fäden 10, 11 und 12 mit den hierdurch entstehenden Netzwaben 13. Zur Kennzeichnung der beiden Hauptdehnungsrichtungen 14 bzw. 15 sind die Fäden 12 bzw. 10 farblich hervorgehoben. Im vorliegenden Beispiel soll die Richtung 15 die stärkere, das heißt unflexiblere, Hauptdehnungsrichtung sein, die Richtung 14 soll der schwächeren Hauptdehnungsrichtung entsprechen. Für den Operateur wird dies durch die Kennzeichnung der Fäden erkennbar: in der hier gezeigten Kennzeichnung entspricht der komplett andersfarbige Faden 10 der stärkeren Hauptdehnungsrichtung 15, während der mit der anderen Farbe gestreifte Faden 12 der schwächeren Hauptdehnungsrichtung 14 entspricht. Das übrige Geflecht besteht aus den einheitlich gefärbten Fäden 11. Zur Hervorhebung der beiden Hauptdehnungsrichtungsfäden 10 bzw. 12 kann die andere verwendete Farbe beispielsweise eine Signalfarbe sein. Die beiden Markierfäden 10 bzw. 12 sollten in regelmäßigen Abständen im Geflecht 9 vorliegen. Hierbei ist es anzustreben, dass die Markierfäden 10 bzw. 12 jeweils in so geringen Abständen parallel verlegt sind, dass auch bei relativ kleinen Implantatstücken im Normalfalle beide Fäden 10, 12 in dem gewählten Ausschnitt enthalten sind.

In Figur 3 ist beispielhaft an einem Bauchverschluss dargestellt, wie die Reparation eines defekten Körperstammes 16 unter Verwendung eines erfindungsgemäßen Implantats 17 bewerkstelligt werden kann. Im Beispiel besteht das Implantat 17 abermals aus einem körperhaften Element 18 und zwei seitlich vorstehenden, an den Kanten 19 befestigten, flächigen Anschlusselementen 20. Insbesondere zur tangentialen Fixierung des Implantats können an den Stellen 21 Nähte zum unter dem Implantat liegenden hinteren Rektusmuskelblatt 27 gesetzt werden. An der Stelle 22 kann darüber hinaus eine weitere Naht gesetzt werden, insbesondere um eine radiale Befestigung des Implantats zu gewährleisten. An den Stellen 23 kann anschließend der strangförmige Teil 18 des Implantats 17 mit den Bauchmuskelvorderblättern 26 mittels Nähten befestigt werden und sodann die Öffnung der Haut 24 an der Stelle 25 zuverlässig verschlossen werden. Deutlich zu erkennen ist in dem Ausführungsbeispiel, wie der körperhafte Teil 18 des Implantats 17 das benachbarte Gewebe 26 zu stützen und dessen Spalte zu füllen vermag. Hierdurch und durch die zusätzliche Befestigung der Muskeln 26 an den Stellen 23 kann erfolgreich vermieden werden, dass an der zu verschließenden Hautstelle 25 eine Klaffung verbleibt. Zudem wird ersichtlich, dass eine mechanische Belastung der Naht 25 kaum noch zu erwarten ist. Die Verwachsung des Implantats 17 mit dem Körpergewebe kann aufgrund der porösen Grundstruktur entlang der gesamten Oberfläche sowohl des körperhaften Elements 18 wie auch der flächenhaften Anschlusselemente 20 erfolgen. Somit kann eine vollständige Integration des Implantats 17 in den Körperstamm auf verträgliche Weise erfolgen.

Selbstverständlich können die gezeigten Ausführungsformen des textilen Implantats auch zumindest teilweise ein resorbierbares Material aufweisen, oder aber auch zumindest teilweise beschichtet sein, ohne dass dies das Beschriebene einschränkt.

## Patentansprüche

1. Textiles Implantat (1), wobei das Implantat monofile Fäden (10. 11, 12) aus Polyvinylidenfluorid (PVDF) aufweist, wobei das Implantat eine netzförmige Flächenstruktur aufweist, wobei das Implantat ein Gewirk, ein Gestrick, ein Gewebe oder ein Gelege aufweist, wobei das Implantat eine poröse und flexible Struktur aufweist, wobei zumindest ein Teil des Implantats ein biokompatibles Material aufweist, wobei an dem Implantat zwei winkelmäßig zueinander versetzt verlaufende Hauptdehnungsrichtungen definiert sind, wobei sich das Implantat bei einer Zugbelastung in einer Hauptdehnungsrichtung bei Fₘₐₓ=16 N/cm um 30 bis 40 % und in einer anderen Hauptrichtung Fₘₐₓ=32 N/cm um 15 bis 25 % dehnt.

2. Textiles Implantat (1) nach Anspruch 1, **dadurch gekennzeichnet, dass** es überwiegend aus monofilen Fäden (10, 11, 12) gebildet ist.

3. Textiles Implantat (1) nach einem der vorhergehenden Ansprüche, **dadurch gekennzeichnet, dass** es eine Porengröße von 1 bis 5 mm, vorzugsweise 1 bis 3 mm, hat.

4. Textiles Implantat (1) nach einem der vorhergehenden Ansprüche, **dadurch gekennzeichnet, dass** zumindest ein Teil des Implantats ein resorbierbares Material aufweist.

5. Textiles Implantat (1) nach einem der vorhergehenden Ansprüche, **dadurch gekennzeichnet, dass** zumindest ein Teil des Implantats eine Beschichtung aufweist.

6. Textiles Implantat nach einem der vorhergehenden Ansprüche, **dadurch gekennzeichnet, dass** zumindest eine der Hauptdehnungsrichtungen durch mindestens einen unterscheidbaren Faden und/oder farblich erkennbar ist.

7. Textiles Implantat nach einem der vorhergehenden Ansprüche, **dadurch gekennzeichnet, dass** es vorstehende Anschlusselemente aufweist.

## Claims

1. Textile implant (1), wherein the implant has monofilament threads (10, 11, 12) of polyvinylidene fluoride (PVDF), wherein the implant has a net-like planar structure, wherein the implant has a formed-loop knit, a drawn-loop knit, a woven fabric or a laid scrim, wherein the implant has a porous and flexible structure, wherein the entire implant has a biocompatible material, wherein two main directions of expansion running angularly offset in relation to each other are defined on the implant, wherein the implant, when subjected to tensile load, expands by 30 to 40% in a main direction of expansion at Fₘₐₓ = 16 N/cm and by 15 to 25% in another main direction at Fₘₐₓ = 32 N/cm.

2. Textile implant (1) according to Claim 1, **characterized in that** it is formed mainly from monofilament threads (10, 11, 12).

3. Textile implant (1) according to either of the preceding claims, **characterized in that** it has a pore size of 1 to 5 mm, preferably 1 to 3 mm.

4. Textile implant (1) according to one of the preceding claims, **characterized in that** at least part of the implant has a resorbable material.

5. Textile implant (1) according to one of the preceding claims, **characterized in that** at least part of the implant has a coating.

6. Textile implant according to one of the preceding claims, **characterized in that** at least one of the main directions of expansion is identifiable by at least one distinguishable thread and/or by colour.

7. Textile implant according to one of the preceding claims, **characterized in that** it has protruding connection elements.

## Revendications

1. Implant textile (1), l'implant présentant des fils en monofilaments (10, 11, 12) en fluorure de polyvinylidène (PVDF), l'implant présentant une structure de surface en forme de filet, l'implant présentant un tissu à mailles, un tricot, un tissu ou une nappe, l'implant présentant une structure poreuse et flexible, l'ensemble de l'implant présentant un matériau biocompatible, deux directions d'étendue principales s'étendant de manière décalée suivant un certain angle l'une par rapport à l'autre étant définies sur l'implant, l'implant, en cas de sollicitation en traction dans une direction d'étendue principale avec Fₘₐₓ = 16 N/cm, s'étirant de 30 à 40 %, et dans une autre direction principale avec Fₘₐₓ = 32 N/cm, s'étirant de 15 à 25 %.

2. Implant textile (1) selon la revendication 1, **caractérisé en ce qu'**il est formé principalement de fils à monofilaments (10, 11, 12).

3. Implant textile (1) selon l'une des revendications précédentes, **caractérisé en ce qu'**il présente une taille de pores de 1 à 5 mm, de préférence de 1 à 3 mm.

4. Implant textile (1) selon l'une quelconque des revendications précédentes, **caractérisé en ce qu'**au moins une partie de l'implant présente un matériau résorbable.

5. Implant textile (1) selon l'une quelconque des revendications précédentes, **caractérisé en ce qu'**au moins une partie de l'implant présente un revêtement.

6. Implant textile selon l'une quelconque des revendications précédentes, **caractérisé en ce qu'**au moins l'une des directions d'étendue principale peut être reconnue au moyen d'un fil distinctif et/ou d'une coloration.

7. Implant textile selon l'une quelconque des revendications précédentes, **caractérisé en ce qu'**il présente des éléments de raccordement saillants.
